# EUROPEAN PATENT APPLICATION

(11) **EP 1 709 975 A1**
(43) Date of publication of application: **11.10.2006**
(21) Application number: 04747538.9
(22) Date of filing: 08.07.2004
(51) Int. Cl.: A61K 47/40, A61K 47/10, A61K 47/26, A61K 47/36, A61K 31/198, A61K 31/375, A61P 3/02

(54) **TABLET AND PROCESS FOR PRODUCING THE SAME**

(30) Priority: 10.07.2003 JP 2003194798
(71) Applicant: KYOWA HAKKO KOGYO CO., LTD., Chiyoda-ku, Tokyo 100-8185 (JP)
(72) Inventor: HARA, Takahiro c/o Kyowa Wellness Co., Ltd., Tokyo 103-8503 (JP); KIMURA, Masao c/o Healthc. Prod. Developm. Center, Tsukuba-shi Ibaraki 305-0841 (JP); SAKAI, Yasushi c/o Healthc. Prod. Developm. Center, Tsukuba-shi Ibaraki 305-0841 (JP)
(74) Representative: Vossius & Partner
(86) International application number: PCT/JP2004/010072
(87) International publication number: WO 2005/004923

(57) **Abstract**

An object of the present invention relates to a tablet which comprises an active ingredient and a cyclodextrin or a cyclodextrin derivative and rapidly disintegrates in the oral cavity, etc. The present invention provides a tablet in which 70% by mass or more of the components of the tablet is a cyclodextrin or a cyclodextrin derivative and a method for manufacturing a tablet comprising the steps of: mixing constituent components of the tablet which comprises as constituent components an active ingredient and a cyclodextrin or a cyclodextrin derivative and in which 70% by mass or more of the total constituent components of the tablet is the cyclodextrin or the cyclodextrin derivative; and subsequently tableting the resultant mixture.

## Description

### Technical Field

The present invention relates to a tablet that rapidly disintegrates in the oral cavity and a method for manufacturing the same.

### Background Art

A tablet has advantages in that it is highly portable, a certain amount can be taken without measuring the dose when it is taken, and the like. However, it has a disadvantage in that it is generally more difficult to be taken compared with a syrup or the like. In particular, in the case of elderly or children whose ability to swallow is weak, many of them avoid taking it for the reasons that it is difficult to swallow, it may get stuck in the pharynx or esophagus and the like. Accordingly, as a tablet that solved such a problem, a tablet that rapidly disintegrates in the oral cavity is known and an intraorally rapid disintegration tablet that can be taken without water is also known.

On the other hand, a cyclodextrin, such as, α-cyclodextrin and β-cyclodextrin or γ-cyclodextrin is a cyclic molecule comprising 6, 7 or 8 glucose units, and its property of solubilizing or stabilizing various compounds has been studied in particular. In addition, various cyclodextrin derivatives are known.

Until now, for example, improvement of the disintegration of a tablet by tableting a powdered cyclodextrin derivative with another diluents (Chemical & Pharmaceutical Bulletin, 32 (2), 665 (1984)), a pharmaceutical composition comprising a slightly soluble drug compound, a cyclodextrin, a physiologically acceptable water-soluble acid and a physiologically acceptable water-soluble organic polymer (Published Japanese translation of a PCT application No. 2002-511073), an adjuvant for direct tableting comprising a diluents for tablet such as a microcrystal cellulose and a binder such as β-cyclodextrin (JP-A-5-339197), a granulated material obtained by spray-coating saccharides composed of a monosaccharide, a disaccharide and a sugar alcohol thereof with a solution comprising a cyclodextrin completely dissolved therein and granulating the resultant material and a compressed tablet derived from the granulated material (JP-A-2002-255796) and the like have been disclosed, and the use of a cyclodextrin as an excipient in the production of a tablet have been known.

### Disclosure of the invention

An object of the present invention is to provide a tablet which comprises a cyclodextrin and rapidly disintegrates in the oral cavity, and a method for manufacturing the same, and the like.

The present invention relates to the following (1) to (35).
(1) A tablet comprising an active ingredient and a cyclodextrin or a cyclodextrin derivative, wherein 70% by mass or more of the components in the tablet is cyclodextrin or the cyclodextrin derivative.
(2) The tablet according to the above-mentioned (1), further comprising a lubricant.
(3) The tablet according to the above-mentioned (2), wherein the lubricant is present only on the surface of the tablet.
(4) The tablet according to any one of the above-mentioned (1) to (3), wherein the tablet is produced by tableting using a punch and/or a die on which a lubricant has been applied.
(5) The tablet according to any one of the above-mentioned (1) to (4), further comprising a saccharide.
(6) The tablet according to the above-mentioned (5), wherein the saccharide is one component or arbitrarily combined plural components selected from the group consisting of a monosaccharide, a disaccharide, a sugar alcohol and an oligosaccharide.
(7) The tablet according to any one of the above-mentioned (1) to (6), further comprising one component or arbitrarily combined plural components selected from the group consisting of a sweetener, an acid, a binder, an antioxidant, a coloring agent, a flavor, a diluent, a fluidizing agent and a disintegrant.
(8) The tablet according to any one of the above-mentioned (1) to (7), wherein the active ingredient is one component or arbitrarily combined plural components selected from the group consisting of a vitamin, a carotenoid, a mineral, an amino acid, an amino acid derivative, an active pharmaceutical ingredient, a plant extract and a health food material.
(9) The tablet according to any one of the above-mentioned (1) to (8), wherein the cyclodextrin is α-cyclodextrin, β-cyclodextrin, maltosyl-β-cyclodextrin or γ-cyclodextrin.
(10) The tablet according to any one of the above-mentioned (1) to (9), which is an intraorally rapid disintegration tablet.
(11) The tablet according to any one of the above-mentioned (1) to (10), which disintegrates in the oral cavity in 40 seconds or less.
(12) The tablet according to any one of the above-mentioned (1) to (11), which has tablet hardness ranging from 25 to 200 N.
(13) A method for manufacturing a tablet comprising an active ingredient and a cyclodextrin or a cyclodextrin derivative, comprising the steps of: mixing constituent components of the tablet which comprises as constituent components an active ingredient and a cyclodextrin or a cyclodextrin derivative and in which the cyclodextrin or the cyclodextrin derivative amounts to 70% by mass or more of the total constituent components; and subsequently tableting the resultant mixture.
(14) The method for manufacturing according to the above-mentioned (13), wherein the tablet further comprises a lubricant.
(15) The method for manufacturing a tablet according to the above-mentioned (14), wherein the mixture does not contain a lubricant and the process further comprises the step of allowing the lubricant to be present only on the surface of the tablet.
(16) The method for manufacturing a tablet according to any one of the above-mentioned (13) to (15), wherein the tableting is carried out using a punch and/or a die on which a lubricant has been applied.
(17) The method for manufacturing a tablet according to any one of the above-mentioned (13) to (16), wherein the mixture further comprises a saccharide.
(18) The method for manufacturing a tablet according to the above-mentioned (17), wherein the saccharide is one component or arbitrarily combined plural components selected from the group consisting of a monosaccharide, a disaccharide, a sugar alcohol and an oligosaccharide.
(19) The method for manufacturing a tablet according to any one of the above-mentioned (13) to (18), wherein the mixture further comprises one component or arbitrarily combined plural components selected from the group consisting of a sweetener, an acid, a binder, an antioxidant, a coloring agent, a flavor, a diluent, a fluidizing agent and a disintegrant.
(20) The method for manufacturing a tablet according to any one of the above-mentioned (13) to (19), wherein the active ingredient is one component or arbitrarily combined plural components selected from the group consisting of a vitamin, a carotenoid, a mineral, an amino acid, an amino acid derivative, an active pharmaceutical ingredient, a plant extract and a health food material.
(21) The method for manufacturing a tablet according to any one of the above-mentioned (13) to (20), wherein the cyclodextrin is α-cyclodextrin, β-cyclodextrin, maltosyl-β-cyclodextrin or γ-cyclodextrin.
(22) The method for manufacturing a tablet according to any one of the above-mentioned (13) to (21), wherein the tablet is an intraorally rapid disintegration tablet.
(23) The method for manufacturing a tablet according to any one of the above-mentioned (13) to (22), wherein the tablet disintegrates in the oral cavity in 40 seconds or less.
(24) The method for manufacturing a tablet according to any one of the above-mentioned (13) to (23), wherein the tablet has tablet hardness ranging from 25 to 200 N.
(25) A method for accelerating disintegration of a tablet comprising an active ingredient and a cyclodextrin or a cyclodextrin derivative characterized by setting the content of the cyclodextrin or the cyclodextrin derivative to 65% by mass or more of the total constituent components of the tablet.
(26) The method for accelerating disintegration of a tablet according to the above-mentioned (25), wherein the tablet further comprises a lubricant as a constituent component.
(27) The method for accelerating disintegration of a tablet according to the above-mentioned (26), which comprises allowing the lubricant to be present only on the surface of the tablet.
(28) The method for accelerating disintegration of a tablet according to any one of the above-mentioned (25) to (27), wherein the tablet is produced by carrying out tableting using a punch and/or a die on which a lubricant has been applied.
(29) The method for accelerating disintegration of a tablet according to any one of the above-mentioned (25) to (28), wherein a saccharide is further comprised as a constituent component of the tablet.
(30) The method for accelerating disintegration of a tablet according to the above-mentioned (29), wherein the saccharide is one component or arbitrarily combined plural components selected from the group consisting of a monosaccharide, a disaccharide, a sugar alcohol and an oligosaccharide.
(31) The method for accelerating disintegration of a tablet according to any one of the above-mentioned (25) to (30), wherein the tablet further comprises as a constituent component one component or arbitrarily combined plural components selected from the group consisting of a sweetener, an acid, a binder, an antioxidant, a coloring agent, a flavor, a diluent, a fluidizing agent and a disintegrant.
(32) The method for accelerating disintegration of a tablet according to any one of the above-mentioned (25) to (31), wherein the active ingredient is one component or arbitrarily combined plural components selected from the group consisting of a vitamin, a carotenoid, a mineral, an amino acid, an amino acid derivative, an active pharmaceutical ingredient, a plant extract and a health food material.
(33) The method for accelerating disintegration of a tablet according to any one of the above-mentioned (25) to (32), wherein the cyclodextrin is α-cyclodextrin, β-cyclodextrin, maltosyl-β-cyclodextrin or γ-cyclodextrin.
(34) The method for accelerating disintegration of a tablet according to any one of the above-mentioned (25) to (33), wherein the tablet is an intraorally rapid disintegration tablet.
(35) The method for accelerating disintegration of a tablet according to any one of the above-mentioned (25) to (34), wherein the tablet has tablet hardness ranging from 25 to 200 N.

The tablet of the present invention, preferably the intraorally rapid disintegration tablet comprises an active ingredient and a cyclodextrin or a cyclodextrin derivative.

Examples of the cyclodextrin include α-cyclodextrin, β-cyclodextrin, maltosyl-β-cyclodextrin, γ-cyclodextrin and the like.

The cyclodextrin derivative is not particularly limited as long as it is a cyclodextrin derivative having the above-mentioned cyclodextrin structure, and examples thereof include derivatives described in WO 98/55148 and the like. Specific examples include derivatives in which one or more hydroxyl groups of a cyclodextrin are substituted with hydrogen, alkyl, hydroxyalkyl, carboxyalkyl, carboxyalkoxyalkyl, alkylcarbonyloxyalkyl, alkoxycarbonylalkyl or hydroxyalkoxyalkyl, and the like.

Here, the alkyl and the alkyl moiety of the alkoxy are preferably an alkyl having 1 to 6 carbon atoms, and preferred examples include methyl, ethyl, 1-methylethyl, 1,1-dimethylethyl, propyl, 2-methylpropyl, butyl, pentyl, hexyl, and the like.

In addition, the cyclodextrin derivative also includes, for example, polyethers as described in U.S. Patent No. 3,459,731, and examples thereof include ethers in which the hydrogen of one or more hydroxyl groups of a cyclodextrin is substituted with an alkyl having 1 to 6 carbon atoms, a hydroxyalkyl having 1 to 6 carbon atoms, a alkyl having 1 to 6 carbon atoms substituted carboxy or a alkyl having 1 to 6 carbon atoms substituted alkyloxycarbonyl having 1 to 6 carbon atoms, or mixed ethers thereof and the like, and preferred examples thereof include ethers substituted with an alkyl having 1 to 3 carbon atoms, a hydroxyalkyl having 2 to 4 carbon atoms, or a alkyl having 1 to 2 carbon atoms substituted carboxy and the like, more preferred examples thereof include ethers substituted with methyl, ethyl, hydroxyethyl, hydroxypropyl, hydroxybutyl, carboxymethyl or carboxyethyl and the like.

In addition, the cyclodextrin derivative also includes, for example, sulfobutylcyclodextrins (U.S. Patent No. 5,134,127) and the like.

Among the cyclodextrins or cyclodextrin derivatives, preferred is α-cyclodextrin, β-cyclodextrin, maltosyl-β-cyclodextrin, γ-cyclodextrin, methyl-β-cyclodextrin, monochlorotriazyl-β-cyclodextrin, hydraxypropyl-β-cyclodextrin or triacetyl-β-cyclodextrin, more preferred is α-cyclodextrin, β-cyclodextrin, maltosyl-β-cyclodextrin or γ-cyclodextrin, and further more preferred is β-cyclodextrin.

The average particle size of the cyclodextrin or the cyclodextrin derivative is not particularly limited, however, for example, it ranges from 1 to 150 µm, preferably from 5 to 100 µm, more preferably from 10 to 80 µm, particularly preferably from 30 to 60 µm. The average particle size can be measured using, for example, a laser diffraction particle distribution measurement apparatus (HEROS & RODOS manufactured by JEOL Ltd.) or the like.

In addition, the density of the cyclodextrin or the cyclodextrin derivative is not particularly limited, however, the density measured based on the volume when a powder is gently placed in a container (loose bulk density) is preferably 0.7 g/mL or less, more preferably 0.6 g/mL or less, particularly preferably 0.5 g/mL or less. The density measured based on the volume obtained by applying vibration (such as tapping) to a container in which a powder is placed until the volume of the powder does not decrease (tapped bulk density) is preferably 0.80 g/mL or less, more preferably 0.75 g/mL or less, particularly preferably 0.70 g/mL or less. The loose and tapped bulk densities can be measured using a measuring device of decrease in apparent specific volume (RHK-type tapping machine, manufactured by KONISHI SEISAKUSHO CO. LTD) or the like. As the tapping condition, for example, a condition where a cylinder is dropped from a distance of 2 cm and the treating time is 1 minute and the like are mentioned.

The water content of the cyclodextrin or the cyclodextrin derivative is preferably 10% by mass or less, more preferably 5% by mass or less.

The compounding ratio of the cyclodextrin and the cyclodextrin derivative in the tablet is 70% by mass or more and less than 100% by mass, and it ranges preferably from 75 to 99.9% by mass, more preferably from 80 to 99.0% by mass and particularly preferably from 85 to 95% by mass.

The cyclodextrin or the cyclodextrin derivative can be used alone or plural types can be used by arbitrarily combining, however, it is preferred that it is used alone.

The active ingredient is not particularly limited, however, examples thereof include a vitamin, a carotenoid, a mineral, an amino acid, an amino acid derivative, an active pharmaceutical ingredient, a plant extract and a health food material and the like.

Examples of the vitamin include vitamin A, vitamin B2, vitamin B1, vitamin B6, vitamin B12, vitamin C, nicotinamide, calcium pantothenate, vitamin K2, vitamin D3, folic acid, pyrroloquinoline quinone and the like.

Examples of the carotenoid include β-carotene, α-carotene, lutein, cryptoxanthin, zeaxanthin, lycopene, astaxanthin, multi-carotene and the like.

Examples of the mineral include calcium, magnesium, dolomite, manganese, zinc, iron, copper, selenium, chromium, sulfur, iodine and the like.

Examples of the amino acid include aliphatic amino acids (specifically glycine, alanine, etc.), branched-chain amino acids (specifically valine, leucine, isoleucine, norleucine, etc.), hydroxyamino acids (specifically serine, threonine, etc.), acidic amino acids (specifically aspartic acid, glutamic acid, etc.), acidic amino acid amides (specifically asparagine, glutamine, etc.), basic amino acids (specifically lysine, hydroxylysine, arginine, ornithine, etc.), sulfur-comprising amino acids (specifically cysteine, cystine, methionine, etc.), aromatic amino acids (specifically phenylalanine, tyrosine, thyronine, etc.), heterocyclic amino acids (specifically tryptophan, histidine, etc.), imino acids (specifically proline, 4-hydroxyproline, etc.) and the like.

Examples of the amino acid derivative include acetylglutamine, acetylcysteine, carboxymethylcysteine, acetyltyrosine, acetylhydroxyproline, 5-hydroxyproline, glutathione, creatine, S-adenylmethionine, glycylglycine, glycylglutamine, dopa, alanylglutamine, carnitine, γ-aminobutyric acid and the like.

Examples of the active pharmaceutical ingredient include aspirin, acetaminophen, ethenzamide, ibuprofen, diphenhydramine, chlorpheniramine, dihydrocodeine, noscapine, methylephedrine, caffeine, serrapeptase, lysozyme, diclofenac sodium, ketoprofen, indometacin, bucolome, pentazocine, chlorpromazine, reserpine, alprazolam, chlordiazepoxide, diazepam, imipramine, maprotiline, estazolam, nitrazepam, diazepam, sodium phenobarbital, scopolamine, papaverine, citicoline, meclofenoxate, phenytoin, carbamazepine, isoproterenol, diastase, lansopraziole, omeprazole, rabrprazole, famotidine, cimetidine, ranitidine, dextromethorphan, guanfacine, codeine, difenidol, metoclopramide, levallorphan, theophylline, salbutamol, amlexanox, seratrodast, oxytetracycline, triamcinolone acetonide, chlorhexidine, lidocaine, diphenhydramine, promethazine, isothipendyl, digoxin, procainamide, propranolol, pindolol, isosorbide, furosemide, delapril, captopril, hydralazine, labetalol, manidipine, candesartan cilexetil, methyldopa, losartan, valsartan, eprosartan, irbesartan, tasosartan, telmisartan, forasartan, phenylephrine, carbocromen, molsidomine, verapamil, simvastatin, pravastatin, trepibutone, cefalexin, amoxicillin, pivmecillinam, cefotiam, cefozopran, cefmenoxime, cefsulodin sodium, ampicillin, ciclacillin, sulbenicillin sodium, nalidixic acid, enoxacin, carumonam sodium, tolbutamide, voglibose, pioglitazone, troglitazone, acarbose, miglitol, emiglitate, ipriflavone, methocarbamol, meclizine, dimenhydrinate, liothyronine sodium, dexamethasone, prednisolone, oxendolone, leuprorelin, opium, morphine, ipecac, oxycodone, opium alkaloid, cocaine, allopurinol, colchicine, 5-fluorouracil, mitomycin and the like.

Examples of the plant extract include aloe, chlorella, prune, propolis, Agaricus blazei, ginseng, ginkgo leaf, kale, Ganoderma, Serenoa repens, turmeric, curcumin, Japanese apricot fruit extract, grape seed, pine resin extract, germinating brown rice, shiitake mushroom mycelium, Rubus suavissimus, Hydrangea serrata, Phellinus linteus, sesame, garlic, champignon, Garcinia cambogia, milk thistle extract, silymarin, St. John's Wort, mulberry leaf, Gymnema sylvestre, Perilla frutescens, Plantago asiatica L., hardy rubber tree tee, oolong tea, Sasa veitchii, guava, Panax notoginseng, citrus, cat's claw, Siberian ginseng, Monascus purpureus, maca, Cordyceps sinensis, Matricaria chamomilla L., chili pepper and the like. In addition, a raw material plant of such a plant extract, its processed product and the like are also included in the plant extract.

Examples of the health food material include royal jelly, dietary fiber, protein, bifidobacteria, lactobacillus, chitosan, health vinegar, yeast, nucleic acid, glucosamine, lecithin, polyphenol, egg yolk oil, phytosterol, docosahexaenoic acid, cartilage derived from an animal, fish or shellfish, soft-shelled turtle, phosphatidyl serine, lactoferrin, freshwater clam, eicosapentaenoic acid, germanium, enzyme, nattokinase, creatine, carnitine, citric acid, raspberry ketone, coenzyme Q10, methyl sulfonyl methane, phospholipid-binding soybean peptide and the like.

The compounding ratio of the active ingredient in the tablet ranges generally from 0.01 to 30% by mass, preferably from 0.05 to 29% by mass, more preferably from 0.1 to 25% by mass, further more preferably from 1 to 20% by mass and particularly preferably from 2 to 15% by mass.

The form of the active ingredient is not particularly limited, however, it is preferably in the form of microcrystalline or microparticulate. The average particle size thereof ranges, for example, from 1 to 100 µm, preferably from 5 to 80 µm, more preferably from 10 to 60 µm and particularly preferably from 30 to 50 µm.

The tablet of the present invention comprises an active ingredient and a cyclodextrin or a cyclodextrin derivative and may contain a pharmaceutical excipient as needed. Examples of the pharmaceutical excipient include a lubricant, a saccharide, a sweetener, an acid, a binder, an antioxidant, a coloring agent, a flavor, a diluent, a fluidizing agent, a disintegrant and the like, and preferred examples thereof include a lubricant, a saccharide, a sweetener, a binder, a diluent, a fluidizing agent, a disintegrant and the like, and more preferred examples thereof include a lubricant, a saccharide, a fluidizing agent and the like. The tablet of the present invention may contain one component or arbitrarily combined plural components of the pharmaceutical excipients.

The lubricant is not particularly limited as long as it can be used in food or the like, and examples thereof include stearic acid, metal salts of stearic acid such as magnesium stearate and calcium stearate, sucrose esters of fatty acids or glycerol esters of fatty acids, hydrogenated castor oil and the like.

The compounding ratio of the lubricant in the tablet ranges preferably from 0 to 20% by mass, more preferably from 0.01 to 10% by mass and particularly preferably from 0.05 to 5% by mass.

In addition, the lubricant may be present only on the surface of the tablet or dispersed in the tablet, however, it is preferred that the lubricant is present only on the surface of the tablet. In this case, the compounding ratio of the lubricant present on the surface ranges preferably from 0.001 to 5% by mass, more preferably from 0.005 to 3% by mass and particularly preferably from 0.01 to 2% by mass.

The saccharide is not particularly limited as long as it can be used in food or the like, however, examples thereof include a monosaccharide, a disaccharide, a sugar alcohol and an oligosaccharide.

Examples of the monosaccharide include glucose, xylose, galactose, fructose and the like. Examples of the disaccharide include trehalose, sucrose, lactose, palatinose and the like. Examples of the sugar alcohol include maltitol, erythritol, sorbitol, xylitol and the like. Examples of the oligosaccharide include raffinose, inulooligosaccharide (oligosaccharide of chicory), palatinose oligosaccharide and the like.

The form of the saccharide is not particularly limited, however, it is preferably in the form of microcrystalline or microparticulate. The average particle size thereof ranges, for example, from 1 to 100 µm, preferably from 5 to 80 µm, more preferably from 10 to 60 µm and particularly preferably from 30 to 50 µm.

The compounding ratio of the saccharide in the tablet ranges preferably from 0 to 29.9% by mass, more preferably from 0.1 to 25% by mass, further more preferably from 1 to 20% by mass and particularly preferably from 2 to 15% by mass.

The sweetener is not particularly limited as long as it can be used in food or the like, however, examples thereof include saccharin sodium, dipotassium glycyrrhizinate, aspartame, stevia, thaumatin, sucralose and the like. The compounding ratio of the sweetener in the tablet is not particularly limited as long as it is within the normal amount of use in a preparation.

The acid is not particularly limited as long as it can be used in food or the like, however, examples thereof include citric acid, tartaric acid, malic acid and the like. The compounding ratio of the acid in the tablet is not particularly limited as long as it is within the normal amount of use in a preparation.

The binder is not particularly limited as long as it can be used in food or the like, however, examples thereof include gelatin, pullulan and the like. The compounding ratio of the binder in the tablet is not particularly limited as long as it is within the normal amount of use in a preparation.

The antioxidant is not particularly limited as long as it can be used in food or the like, however, examples thereof include tocopherol, cysteine hydrochloride, L-ascorbate stearate esters and the like. The compounding ratio of the antioxidant in the tablet is not particularly limited as long as it is within the normal amount of use in a preparation.

The coloring agent is not particularly limited as long as it can be used in food or the like, however, examples thereof include Yellow #5 dye for food, Red #2 dye for food, Blue #2 dye for food and the like. The compounding ratio of the coloring agent in the tablet is not particularly limited as long as it is within the normal amount of use in a preparation.

The flavor is not particularly limited as long as it can be used in food or the like, however, examples thereof include lemon flavor, lemon lime flavor, grapefruit flavor, apple flavor, orange flavor and the like. The compounding ratio of the flavor in the tablet is not particularly limited as long as it is within the normal amount of use in a preparation.

The diluent is not particularly limited as long as it can be used in food or the like, however, examples thereof include microcrystalline cellulose, low substituted hydroxypropylcellulose and the like. The compounding ratio of the diluent in the tablet ranges preferably from 0 to 25% by mass, more preferably from 0.01 to 20% by mass, further more preferably from 0.1 to 15% by mass and particularly preferably from 1 to 10% by mass.

The fluidizing agent is not particularly limited as long as it can be used in food or the like, however, examples thereof include calcium phosphate, calcium hydrogen phosphate, microfine silicon dioxide and the like. The compounding ratio of the fluidizing agent in the tablet ranges preferably from 0 to 20% by mass, more preferably from 0.01 to 10% by mass and particularly preferably from 0.05 to 5% by mass.

The disintegrant is not particularly limited as long as it can be used in food or the like, however, examples thereof include corn starch, potato starch, partly pregelatinized starch and the like. The compounding ratio of the disintegrant in the tablet ranges preferably from 0 to 25% by mass, more preferably from 0.01 to 20% by mass, further more preferably from 0.1 to 15% by mass and particularly preferably from 1 to 10% by mass.

The tablet of the present invention preferably has a hardness such that, for example, the tablet does not have a chip or does not crumble. The tablet hardness is measured by using generally a tablet hardness tester as a breaking strength in the diametrical direction of the tablet, and the value ranges preferably from 15 to 300 N, more preferably from 25 to 200 N, particularly preferably from 40 to 100 N. The tablet hardness can be measured by using a commercially available measuring device of tablet breaking strength such as TH-203CP manufactured by TOYAMA SANGYO Co., Ltd.

The tablet of the present invention is preferably an intraorally rapid disintegration tablet with rapid disintegration in the oral cavity that rapidly disintegrates when exposed to saliva in the oral cavity without chewing. The disintegration time in the oral cavity is preferably 60 seconds or less, more preferably 40 seconds or less, further more preferably 30 seconds or less and particularly preferably 20 seconds or less.

The disintegration time in the oral cavity can be obtained as follows. The time needed for the tablet to disintegrate when, for example, plural healthy adults such as 5 adults sublingually take the tablet without chewing (time that elapses before feeling remains in the oral cavity disappears, or before a person can swallow the tablet without water or chewing) is measured more than once, for example 5 times, and the disintegration time is calculated as a mean value of these measurement values.

The tablet of the present invention can be taken with water and/or by chewing according to the preference of a person who takes the tablet, however, it can be taken with a small amount of water that can wet the oral cavity or without water.

The shape of the tablet of the present invention is not particularly limited, however, for example, a round tablet, a triangular tablet, a cannonball tablet and the like can be mentioned. The size of the tablet of the present invention is not particularly limited, however, it is preferred that the mass ranges from 0.1 to 2 g and the diameter ranges from 0.3 to 2.0 cm.

The tablet of the present invention can be produced by, for example, a method for manufacturing comprising the steps of: mixing all the above-mentioned constituent components of the tablet in the state of powder or granulating part of the constituent components followed by mixing it with the rest of the constituent components, or granulating all the constituent components; and subsequently compression molding the resultant mixture or granulated material to produce a tablet. More specifically, it can be produced by a process in which an active ingredient and a cyclodextrin or a cyclodextrin derivative and, as needed, one component or arbitrarily combined plural components selected from the group consisting of a lubricant, a saccharide, a sweetener, an acid, a binder, an antioxidant, a coloring agent, a flavor, a diluent, a fluidizing agent and a disintegrant are mixed and the resultant mixture is compression molded by a direct tableting method, or a method for manufacturing in which part of the respective constituent components of the tablet is granulated and mixed with the rest of the components or all the constituent components are granulated and the resultant mixture or granulated material is compression molded (a method for manufacturing of compression molding by an indirect tableting method). The apparatus to be used for compression molding is not particularly limited, and a compression machine such as a rotary compression molding machine and a hydraulic press machine can be used. The above-mentioned method for manufacturing in which compression molding is carried out by a direct tableting method is an extremely convenient method for manufacturing because the respective constituent components of the tablet are only mixed and subjected to compression molding, therefore it is preferred, and water or the like is not added to the constituent components during the method for manufacturing, therefore it is also preferred in terms of the stability of the constituent components of the tablet.

In addition, the tablet of the present invention can also be produced by mixing a lubricant with the above-mentioned mixture or granulated material, however, it can also be produced by a so-called externally lubricating tableting method, in which an extremely small amount of a lubricant is applied on a punch and/or a die of a compression molding machine in advance and the mixture or the granulated material which does not contain a lubricant is compression molded using the compression molding machine having the punch and/or the die on which the lubricant has been applied. Since the amount of the lubricant used is extremely small, this method is preferred in terms of the disintegration after taking the tablet or the stability of the constituent components of the tablet. More specifically, an active ingredient and a cyclodextrin or a cyclodextrin derivative, and as needed, one component or arbitrarily combined plural components selected from the group consisting of a saccharide, a sweetener, an acid, a binder, an antioxidant, a coloring agent, a flavor, a diluent, a fluidizing agent and a disintegrant are mixed to obtain a mixture without adding a lubricant and the resultant mixture is directly tableted using a compression machine such as a rotary tableting machine or a hydraulic press machine having a punch and/or a die on which a lubricant has been applied, whereby the tablet of the present invention can be obtained. The method of applying a lubricant on a punch and/or a die is not particularly limited as long as it is a method of achieving a condition in which a lubricant has been applied on a punch and/or a die, however, for example, a method in which a lubricant powder or a solution comprising a lubricant is sprayed by compressed air or the like, a method in which a lubricant powder or a lubricant is applied with an applicator, a brush or the like, a method in which a punch and/or a die is dipped in a lubricant powder or a solution comprising a lubricant, a method in which a lubricant powder is tableted and the like can be mentioned.

As the granulation method in the case of granulating part of or all the constituent components of the tablet of the present invention (for example, the above-mentioned process in which compression molding is carried out by an indirect tableting method, etc.), a wet-granulation method using purified water, ethanol or the like, a dry-granulation method and the like can be mentioned. The apparatus to be used for granulation is not particularly limited, and for example, a fluidized bed granulator, an agitation granulator, an extrusion granulator or the like can be used.

The tableting pressure at the time of tableting is not particularly limited, however, it is optimally set so as to give a desired hardness of tablet and disintegration time. It is set so as to give a tablet hardness ranging preferably from 15 to 300 N, more preferably from 25 to 200 N, particularly preferably from 40 to 100 N, and to give disintegration time in the oral cavity of preferably 60 seconds or less and more preferably 40 seconds or less, further more preferably 30 seconds or less, particularly preferably 20 seconds or less.

In addition, in the tablet comprising an active ingredient and a cyclodextrin or a cyclodextrin derivative, by setting the compounding ratio of the cyclodextrin or the cyclodextrin derivative to 65% by mass or more of the total constituent components of the tablet, the disintegration of the tablet comprising the active ingredient and the cyclodextrin or the cyclodextrin derivative, preferably the disintegration in the oral cavity can be accelerated. For example, in the above-mentioned description relating to the tablet and the method for manufacturing of the tablet of the present invention, by setting the compounding ratio of the cyclodextrin or the cyclodextrin derivative in the tablet to 65% by mass or more, a method of the present invention for accelerating the disintegration of the tablet comprising the active ingredient and the cyclodextrin or the cyclodextrin derivative, preferably the disintegration in the oral cavity can be implemented.

### Brief Description of the Drawing

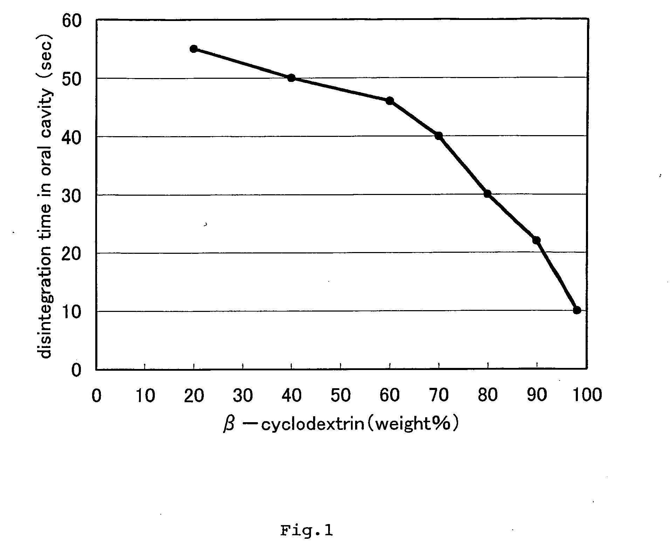

Fig. 1 shows the correlation between the content of β-cyclodextrin in a tablet (% by mass) and disintegration time (second) of the tablet in the oral cavity.

### Best Mode for Carrying Out the Invention

Hereinafter, specific Examples will be described.

### Example 1

After β-cyclodextrin (CELDEX-B-100, manufactured by NIHON SHOKUHIN KAKO CO., LTD) with an average particle size of 49 µm, a loose bulk density of 0.46 g/mL and a tapped bulk density of 0.72 g/mL and maltose (Sunmalt S, manufactured by Hayashibara) were fully mixed in a polyethylene bag at a compounding ratio shown in Table 1, sucrose ester of fatty acid (DK ESTER F20W, manufactured by DAI-ICHI KOGYO SEIYAKU CO., LTD.) as a lubricant was applied on a punch and a die, and a tablet with a diameter of 15 mm and a weight of 750 mg was tableted using a single tableting machine (vertical tableting machine 6B-2M, manufactured by KIKUSUI SEISAKUSHO LTD). The tableting pressure was adjusted so that the tablet harness becomes 49 N each.

The tablet hardness was measured using a KHT-20N hardness tester (manufactured by FUJIWARA SCIENTIFIC CO., LTD).

**Table 1**

| | | | | | | | |
|---|---|---|---|---|---|---|---|
| β-cyclodextrin (% by mass) | 20 | 40 | 60 | 70 | 80 | 90 | 98 |
| Maltose (% by mass) | 80 | 60 | 40 | 30 | 20 | 10 | 2 |

The correlation between the content of β-cyclodextrin in a tablet (% by mass) and disintegration time (second) in the oral cavity of the tablet is shown in Fig. 1. As shown in Fig. 1, the disintegration time of each tablet obtained in Example 1 decreased linearly little by little as the content of β-cyclodextrin increased to the point where the compounding ratio of β-cyclodextrin and maltose is 60:40, but from the ratio of 70:30 it decreased significantly as the content of β-cyclodextrin increased. In other words, it is found that a tablet comprising the cyclodextrin comes to have rapid disintegration in the oral cavity by setting the content of the cyclodextrin to 65% or more.

### Example 2

A vitamin C-comprising tablet A comprising 71.33 % by mass of β-cyclodextrin was produced in the same manner as in Example 1 except that mixing was carried out according to the formulation of Composition A shown in Table 2 and after mixing, the tableting pressure was adjusted so that the tablet hardness becomes 29.4 N. β-cyclodextrin (CELDEX-B-100, manufactured by NIHON SHOKUHIN KAKO CO., LTD), lactose (SUPER-TAB, manufactured by ASAHI KASEI CORPORATION), vitamin C (manufactured by ROCHE VITAMINS JAPAN K. K.), calcium hydrogen phosphate (manufactured by TAIHEI CHEMICAL INDUSTRIAL CO., LTD.), orange flavor (manufactured by T. HASEGAWA CO., LTD.), sucralose (manufactured by SAN-EI GEN F.F.I., Inc.) and sucrose ester (DK ESTER F20W, manufactured by DAI-ICHI KYOGO SEIYAKU CO., LTD.) were used.

**Table 2**

| Formulation | Composition A (% by mass) | Composition B (% by mass) |
|---|---|---|
| β-cyclodextrin | 71.33 | 0 |
| Partly pregelatinized starch | 0 | 71.33 |
| Lactose | 24.50 | 24.50 |
| Vitamin C | 3.06 | 3.06 |
| Calcium hydrogen phosphate | 1.02 | 1.02 |
| Orange flavor | 0.10 | 0.10 |
| Scralose | 0.03 | 0.03 |

### Comparative Example 1

A vitamin C-comprising tablet B comprising 71.33% by mass of partly pregelatinized starch was produced in the same manner as in Example 1 except that mixing was carried out according to the formulation of Composition B shown in Table 2 and after mixing, the tableting pressure was adjusted so that the tablet hardness becomes 29.4 N. Partly pregelatinized starch (PCS FC-30, manufactured by ASAHI KASEI CORPORATION), lactose (SUPER-TAB, manufactured by ASAHI KASEI CORPORATION), vitamin C (manufactured by ROCHE VITAMINS JAPAN K. K.), calcium hydrogen phosphate (manufactured by TAIHEI CHEMICAL INDUSTRIAL CO., LTD.), orange flavor (manufactured by T. HASEGAWA CO., LTD.), sucralose (manufactured by SAN-EI GEN F.F.I., Inc.) and sucrose ester (DK ESTER F20W, manufactured by DAI-ICHI KYOGO SEIYAKU CO., LTD.) were used.

### Example 3

The disintegration time in the oral cavity (time required for a tablet to be disintegrated so that feeling remains in the oral cavity is disappeared, or time required for a person to feel that a tablet has disintegrated so that the person could swallow the tablet without water or chewing) of the tablet A produced in Example 2 and the tablet B produced in Comparative Example 1 was measured. The measurement was carried out with 7 normal healthy subjects.

**Table 3**

| Tablet | Hardness | Disintegration time in the oral cavity |
|---|---|---|
| Tablet A in Example 2 | 29.4 N | 15 seconds |
| Tablet B in Comparative example 1 | 29.4 N | 120 seconds |

As shown in Table 3, the tablet A obtained in Example 2 showed rapid disintegration in the oral cavity than the tablet B obtained in Comparative Example 1.

### Industrial Applicability

According to the present invention, a tablet that rapidly disintegrates in the oral cavity and a process for producing the same and the like are provided.

## Claims

1. A tablet comprising an active ingredient and a cyclodextrin or a cyclodextrin derivative, wherein 70% by mass or more of the components in the tablet is cyclodextrin or the cyclodextrin derivative.

2. The tablet according to Claim 1, further comprising a lubricant.

3. The tablet according to Claim 2, wherein the lubricant is present only on the surface of the tablet.

4. The tablet according to any one of Claims 1 to 3, wherein the tablet is produced by tableting using a punch and/or a die on which a lubricant has been applied.

5. The tablet according to any one of Claims 1 to 4, further comprising a saccharide.

6. The tablet according to Claim 5, wherein the saccharide is one component or arbitrarily combined plural components selected from the group consisting of a monosaccharide, a disaccharide, a sugar alcohol and an oligosaccharide.

7. The tablet according to any one of Claims 1 to 6, further comprising one component or arbitrarily combined plural components selected from the group consisting of a sweetener, an acid, a binder, an antioxidant, a coloring agent, a flavor, a diluent, a fluidizing agent and a disintegrant.

8. The tablet according to any one of Claims 1 to 7, wherein the active ingredient is one component or arbitrarily combined plural components selected from the group consisting of a vitamin, a carotenoid, a mineral, an amino acid, an amino acid derivative, an active pharmaceutical ingredient, a plant extract and a health food material.

9. The tablet according to any one of Claims 1 to 8, wherein the cyclodextrin is α-cyclodextrin, β-cyclodextrin, maltosyl-β-cyclodextrin or γ-cyclodextrin.

10. The tablet according to any one of Claims 1 to 9, which is an intraorally rapid disintegration tablet.

11. The tablet according to any one of Claims 1 to 10, which disintegrates in the oral cavity in 40 seconds or less.

12. The tablet according to any one of Claims 1 to 11, which has tablet hardness ranging from 25 to 200 N.

13. A method for manufacturing a tablet comprising an active ingredient and a cyclodextrin or a cyclodextrin derivative, comprising the steps of: mixing constituent components of the tablet which comprises as constituent components an active ingredient and a cyclodextrin or a cyclodextrin derivative and in which the cyclodextrin or the cyclodextrin derivative amounts to 70% by mass or more of the total constituent components; and subsequently tableting the resultant mixture.

14. The method for manufacturing according to Claim 13, wherein the tablet further comprises a lubricant.

15. The method for manufacturing a tablet according to Claim 14, wherein that the mixture does not contain a lubricant and the process further comprises the step of allowing the lubricant to be present only on the surface of the tablet.

16. The method for manufacturing a tablet according to any one of Claims 13 to 15, wherein the tableting is carried out using a punch and/or a die on which a lubricant has been applied.

17. The method for manufacturing a tablet according to any one of Claims 13 to 16, wherein the mixture further comprises a saccharide.

18. The method for manufacturing a tablet according to Claim 17, wherein the saccharide is one component or arbitrarily combined plural components selected from the group consisting of a monosaccharide, a disaccharide, a sugar alcohol and an oligosaccharide.

19. The method for manufacturing a tablet according to any one of Claims 13 to 18, wherein the mixture further comprises one component or arbitrarily combined plural components selected from the group consisting of a sweetener, an acid, a binder, an antioxidant, a coloring agent, a flavor, a diluent, a fluidizing agent and a disintegrant.

20. The method for manufacturing a tablet according to any one of Claims 13 to 19, wherein the active ingredient is one component or arbitrarily combined plural components selected from the group consisting of a vitamin, a carotenoid, a mineral, an amino acid, an amino acid derivative, an active pharmaceutical ingredient, a plant extract and a health food material.

21. The method for manufacturing a tablet according to any one of Claims 13 to 20, wherein the cyclodextrin is α-cyclodextrin, β-cyclodextrin, maltosyl-β-cyclodextrin or γ-cyclodextrin.

22. The method for manufacturing a tablet according to any one of Claims 13 to 21, wherein the tablet is an intraorally rapid disintegration tablet.

23. The method for manufacturing a tablet according to any one of Claims 13 to 22, wherein the tablet disintegrates in the oral cavity in 40 seconds or less.

24. The method for manufacturing a tablet according to any one of Claims 13 to 23, wherein the tablet has a tablet hardness ranging from 25 to 200 N.

25. A method for accelerating disintegration of a tablet comprising an active ingredient and a cyclodextrin or a cyclodextrin derivative **characterized by** setting the content of the cyclodextrin or the cyclodextrin derivative to 65% by mass or more of the total constituent components of the tablet.

26. The method for accelerating disintegration of a tablet according to Claim 25, wherein the tablet further comprises a lubricant as a constituent component.

27. The method for accelerating disintegration of a tablet according to Claim 26, which comprises allowing the lubricant to be present only on the surface of the tablet.

28. The method for accelerating disintegration of a tablet according to any one of Claims 25 to 27, wherein the tablet is produced by carrying out tableting using a punch and/or a die on which a lubricant has been applied.

29. The method for accelerating disintegration of a tablet according to any one of Claims 25 to 28, wherein a saccharide is further comprised as a constituent component of the tablet.

30. The method for accelerating disintegration of a tablet according to Claim 29, wherein the saccharide is one component or arbitrarily combined plural components selected from the group consisting of a monosaccharide, a disaccharide, a sugar alcohol and an oligosaccharide.

31. The method for accelerating disintegration of a tablet according to any one of Claims 25 to 30, wherein the tablet further comprises as a constituent component one component or arbitrarily combined plural components selected from the group consisting of a sweetener, an acid, a binder, an antioxidant, a coloring agent, a flavor, a diluent, a fluidizing agent and a disintegrant.

32. The method for accelerating disintegration of a tablet according to any one of Claims 25 to 31, wherein the active ingredient is one component or arbitrarily combined plural components selected from the group consisting of a vitamin, a carotenoid, a mineral, an amino acid, an amino acid derivative, an active pharmaceutical ingredient, a plant extract and a health food material.

33. The method for accelerating disintegration of a tablet according to any one of Claims 25 to 32, wherein the cyclodextrin is α-cyclodextrin, β-cyclodextrin, maltosyl-β-cyclodextrin or γ-cyclodextrin.

34. The method for accelerating disintegration of a tablet according to any one of Claims 25 to 33, wherein the tablet is an intraorally rapid disintegration tablet.

35. The method for accelerating disintegration of a tablet according to any one of Claims 25 to 34, wherein the tablet has tablet hardness ranging from 25 to 200 N.
